# EUROPEAN PATENT SPECIFICATION

(11) **EP 2 066 568 B1**
(45) Date of publication and mention of the grant of the patent: **03.10.2012**
(21) Application number: 07803596.1
(22) Date of filing: 25.09.2007
(51) Int. Cl.: B65B 55/19, A61L 2/20, B65D 51/00, B65D 77/00, B65D 81/26

(54) **PROCESS FOR PROVIDING AN OXYGEN-FREE ATMOSPHERE WITHIN A CONTAINER**
VERFAHREN ZUR BEREITSTELLUNG EINER SAUERSTOFFFREIEN ATMOSPHÄRE IN EINEM BEHÄLTER
PROCÉDÉ PERMETTANT DE CRÉER UNE ATMOSPHÈRE DÉPOURVUE D'OXYGÈNE À L'INTÉRIEUR D'UN RÉCIPIENT

(30) Priority: 27.09.2006 GB 0619060
(43) Date of publication of application: 10.06.2009
(73) Proprietor: Aseptic Technologies S.A., 5032 Les Isnes (BE)
(72) Inventor: BALERIAUX, Patrick, 1330 Rixensart (BE); THILLY, Jacques, 1330 Rixensart (BE)
(74) Representative: Luys, Marie-José A.H.
(86) International application number: PCT/EP2007/060134
(87) International publication number: WO 2008/037699

(56) References cited:
- EP-A- 0 167 229
- EP-A- 1 520 795
- WO-A-03/039632
- GB-A- 656 392
- GB-A- 2 208 287
- JP-A- 8 164 185
- JP-A- 62 138 438
- JP-A- 2002 065 808
- JP-A- 2003 292 055
- US-A- 4 645 073
- US-A- 4 872 553
- US-A- 6 007 529
- US-A1- 2003 106 824
- US-B1- 6 183 790
- US-B1- 6 877 601

## Description

This invention relates to a novel process for providing containers with a reduced interior level of oxygen.

In particular the invention relates to containers suitable for containing medicinal and cosmetic substances. The term "medicinal substance" herein is intended to include any type of prophylactic or therapeutic pharmaceutical compounds and formulations and vaccines, or reconstitution media, whether for human and veterinary administration, whether liquid or solid, for reconstitution or for administration without reconstitution. Otherwise the invention may be used for containers for the containment of any kind of oxygen-sensitive material.

Various types of containers are commonly used for the containment of medicinal substances. Examples of such containers include, without limitation, vials, syringes, capsules and carpules.

Such containers for the containment of medicinal substances are often made of glass but more recently have been made of plastics materials, in particular cycloelefin copolymer ("COC"), a blend thereof or a blend thereof with another polymer. Examples of such COC polymers are for example disclosed in US-A-5723189, EP-A-0436372 and EP-A-0556034 among others. A COC plastic material accepted for use in the pharmaceutical industry is the cyclolefin copolymer "Topas" made by Topas Advanced Polymers, for example the known COC polymers Topas 8007, Topas 6013 or Topas 6015 available from for example Topas Advanced Polymers. Cyclo olefin polymers are also available from the company Zeon.

Vials for the containment of medicinal substances generally have open mouths which are normally closed with an elastomer closure through which a hollow needle may be passed and via which liquid may be introduced or removed from the vial. Suitable elastomers for such closures are well known and may be based on styrenic block copolymer thermoplastic elastomers as alternative to vulcanized elastomers commonly used for vial closures. One such elastomer material is that disclosed in WO-A-2005/014419.

Syringes generally comprise a cylindrical barrel, often made of glass but more recently have been made of plastics materials, for example the COC polymers mentioned above. The barrels of such syringes are normally closed with an elastomer plunger which can be urged along the barrel to eject liquid content via a nozzle. Suitable elastomers for such plungers may be based on the same thermoplastic elastomers as mentioned above for vial closures.

Often such vials are filled with medicinal substance by introducing the substance via the open mouth, then closing the mouth with the closure, optionally after lyophilising the substance. Syringes are generally filled by introducing the substance into the open end of the barrel opposite the nozzle, then closing this open end by inserting the plunger.

An alternative filling procedure, termed herein "sterile filling", has been developed for containers in which a container is provided, having a sterile interior and a puncturable wall part, a hollow filling needle is passed through the puncturable part of the container wall, a liquid material content is introduced via the needle, the needle is withdrawn, and the filing needle is then withdrawn. The residual puncture hole is then closed e.g. by the elasticity of the wall material, by a cover or by heat sealing, for example see US-A-5,641,004. For the sterile filling of vials, conveniently vials are provided with a sterile interior and their mouths closed with a closure, and are filled by passing a hollow filling needle through the closure, and introducing a liquid medicinal substance therethrough. In an analogous procedure syringes are provided with a sterile interior and with their open end closed with the plunger and/or with their opposite nozzle end closed with a puncturable closure, and are filled by passing a hollow filling needle through the plunger or closure, and then introducing a liquid medicinal substance therethrough. The filling needle is then withdrawn and the elasticity of the elastomer closure or plunger closes the residual puncture hole. The residual puncture hole may then be more completely sealed by locally heating the closure in the vicinity of the puncture hole e.g. with a focused laser beam. A vial or syringe filled in this way is termed herein a "sterile filled vial" or "sterile filled syringe". This procedure is for example disclosed in WO-A-2002064439, WO-A-. 200407187, WO-A-2004026695, WO-A-2004026735, WO-A-2004076288 disclosing combination according to the preamble of claim 13, WO-A-2005086677, WO-A-2005046755, WO-A-2006/058786 and EP-A-0679574.

Methods of making vials suitable for such a sterile filling process by for example simultaneous moulding then assembly of separate vials and closures are disclosed in WO-A-2005005128. A particular type of vial suitable for use in such a process is that disclosed in WO-A-2004/018317, which is preferably made of the above-mentioned COC polymer and closure made of the above-mentioned elastomers.

Examples of medicinal substances contained within such containers include any type of prophylactic or therapeutic pharmaceutical compounds and formulations and vaccines, or reconstitution media, whether for human and veterinary administration, whether liquid or solid, for reconstitution or for administration without reconstitution. As mentioned above.

A common problem with medicinal substances is their vulnerability to atmospheric oxygen, which can cause oxidative degradation of such substances. The materials used for the envelopes of containers of medicinal substances are often permeable to atmospheric oxygen. Ways of preventing access of atmospheric oxygen to medicinal substances contained in various types of container are known.

One known solution to the problem of atmospheric oxygen is to provide an oxygen-free atmosphere within the container such as a vial or syringe prior to introducing the medicinal substance content. For example this may be achieved by flushing the open container such as a vial with nitrogen just before it is filled, and to provide a second flush of nitrogen after filling, to further secure a low oxygen concentration in the headspace before stoppering. This is relatively straightforward in the case of vials which are filled via their open mouths before insertion of a closure. But in the case of the above-mentioned sterile filling procedure in which the vial is filled with its closure already in place this results in the problem that the vials or syringes need to be fitted with their closures and plungers under an oxygen-free atmosphere. The need for such an oxygen-free atmosphere covering the assembly machinery can be a considerable inconvenience and expense. For example in the process disclosed in WO-A-2005005128 relatively large assembly robots are required.

To protect filled containers against long term oxidation by atmospheric oxidation during storage, it is also known to enclose the closed container containing the medicinal substance, i.e. after filling, within an outer envelope, and to locate an oxygen-absorbing material in the space between the container and the outer envelope. Examples of the many disclosures of this are GB-A-2 208 287, JP-A-2002065808, US-A-2003/0106824, US-A-4,872,553, US-A-6,007,529 and WO-A-03039632. Numerous types of oxygen-absorbing material are known, for example iron and iron oxides.

Therefore there is an ongoing need to find a solution to the problem of providing an oxygen-free atmosphere within a container, particularly vials and syringes to be filled by the above-mentioned sterile filling procedure. It is an object of this invention to provide such a solution. Other objects and advantages of the present invention will be apparent from the following description.

According to this invention a process for the provision of a closed container having an internal atmosphere containing a lower concentration of oxygen than ambient atmospheric is provided, according to claim 1.

The present invention works by exploiting the oxygen-permeability of the container wall and/or closure material. The oxygen-absorbing material absorbs oxygen from the space between the container and the envelope wall. Consequently oxygen diffuses out of the container into this space to be absorbed by the oxygen-absorbing material, and other atmosphere gases such as nitrogen etc. diffuse in the opposite direction through the container wall and/or closure material into the container to replace the oxygen which has diffused out.

Ideally ultimately all of the oxygen within the container may be removed and replaced by these other atmosphere gases. In practice it is found that the process of the invention can reduce the concentration of oxygen in the atmosphere within the container to 1% or less. This is generally adequate to minimize to an acceptable extent any oxidative damage to medicinal materials within the container.

The boundary may be made of an oxygen-permeable puncturable material. Additionally or alternatively the boundary may comprise a boundary wall having an opening therethrough into the interior of the container and which is closed by an oxygen-permeable closure material, preferably a puncturable closure material.

The container may be a vial comprising a vial body bounded by a body wall and having a mouth closed by a puncturable vial closure.

Alternatively the container may be a syringe barrel having a nozzle end and an open end opposite its nozzle end, bounded by a barrel wall and closed by a puncturable plunger. Alternatively there may be an opening at the nozzle end of the syringe, and closed by an oxygen-permeable puncturable closure.

Suitable vials and syringes may have a vial body wall or barrel wall which is made of an oxygen-permeable plastics material. In particular the above-mentioned COC polymer, a blend thereof or a blend thereof with another polymer, such as "Topas", e.g. Topas 8007, 6015 or 6013 may be used.

Vials and syringes may have closures or plungers made of an oxygen-permeable material, with a vial body or barrel wall made of an oxygen-permeable material such as the above-mentioned COC polymers, or of an oxygen-impermeable material such as oxygen-impermeable glass. A suitable oxygen-permeable material is an elastomer material, many of which have a known permeability to oxygen, for example the elastomer disclosed in WO-A-2005/014419 It is found that when made of such materials vials, syringes, closures and plungers of conventional dimensions as used for vials and syringes have a suitable oxygen permeability for use in this invention. Such a vial may be bounded by a vial body wall made of a COC polymer and have a mouth opening which is closed by a puncturable closure.

Suitably the container is empty of liquid content.

The container has an internal volume of which 50% or more is initially an oxygen-containing atmosphere. Preferably the container has an internal volume of which 75% or more is initially an oxygen-containing atmosphere. More preferably the container has an internal volume of which 90% or more is initially an oxygen-containing atmosphere. Most preferably the container is empty except for this initial oxygen-containing atmosphere. Alternatively the container may contain an additive for mixing with a liquid substance to be introduced into the container.

In such a state the container is suitable for filling by means of the above-mentioned sterile filling process. For example vials made by the process as disclosed in WO-A-2005005128 of simultaneously moulding vials and closures then fitting closures to vials are made in such an empty state. Such vials are normally made in an atmosphere of filtered atmospheric air and consequently will contain an oxygen-containing atmosphere i.e. air.

Containers of this type, being vials having a vial wall made of a COC polymer and having a mouth opening closed by a closure made of an oxygen-permeable elastomer and having a sterile interior are for example commercially available from the company Aseptic Technologies, Les Isnes, Belgium.

The envelope itself may enclose an atmosphere with an oxygen concentration less than atmospheric. For example prior to closing the envelope around one or plural containers air in the envelope may be replaced with an inert gas such as nitrogen. In such a process the oxygen absorbing material absorbs substantially only the oxygen diffusing out of the one or plural containers within the envelope.

The envelope wall should be as airtight as possible, and may suitably be a flexible wall to accommodate to the reduction of volume of ca. 20% as the oxygen is absorbed from the atmosphere between the envelope wall and the container therein, the ambient concentration of oxygen in the air being ca. 20% by volume. The air atmosphere inside the envelope may be replaced by an oxygen-reduced atmosphere e.g. nitrogen, prior to sealing the envelope around the container, but commercially available oxygen absorbing materials are capable of absorbing the ca 20% of oxygen within the air in a typical envelope. Alternatively or additionally the envelope may be fed with a supply of a non-oxygen gas such as nitrogen to make up the volume as the oxygen is used up, Few materials are 100% impermeable to oxygen, but many have a sufficient degree of impermeability that when used in combination with an oxygen-absorbing material an effectively low level of oxygen can be achieved within the envelope. Suitable flexible envelope wall materials are known in the art, for example aluminium foil or aluminium foil - plastics material laminates. Examples of suitable flexible materials for such an envelope wall are known, for example disclosed in WO-A-200303962. A suitable envelope material comprises aluminium foil, typically with a minimum thickness of 7 microns, preferably 9-12 microns, laminated with polymer to protect the aluminium and to facilitate a heat seal of the envelope.

Suitably the volume of the space between the container and the envelope wall should be minimised to facilitate the rapid removal of atmospheric oxygen therefrom.

Numerous oxygen-absorbing materials are known in the art, which are suitable for use in the pharmaceutical, vaccine and other medicinal product industries. Some oxygen-absorbing materials are able to reduce atmospheric oxygen levels to as little as 0.1% and such a level within the envelope is suitable for many uses of the invention. Many such oxygen-absorbing materials are based upon iron particles which oxidise on exposure to atmospheric oxygen to chemically bind oxygen and thereby remove it from the atmosphere. Some oxygen-absorbing material require moisture to function, some do not. Some oxygen-absorbing materials also absorb moisture from the atmosphere whilst absorbing oxygen, some do not. Some oxygen-absorbing materials liberate non-oxygen gases such as carbon dioxide into the atmosphere whilst they absorb oxygen. Some do not. The choice of which oxygen-absorbing material to use may depend upon the application of the invention. A suitable oxygen-absorbing material, suitable for medical applications and not requiring the presence of moisture to function, is commercially available from Multisorb, being a proprietary and undisclosed formulation.

The process conditions and the length of time a container such as a vial or syringe needs to be enclosed within the envelope to achieve a desired level of oxygen concentration inside the container will depend upon inter-alia the material and dimensions of the container and closure or plunger, the volume and characteristics of the envelope, and the oxygen-absorbing material. Many known oxygen-absorbing materials operate at ambient temperatures.

Suitably after the oxygen concentration within the container has reached the predetermined level below ambient atmospheric concentration as a result of the process of this invention, the container may be removed from the envelope for further processing.

To preserve the oxygen concentration within the container below ambient atmospheric concentration the container may be stored in advance of further processing within the envelope, or else in another storage environment with a low atmospheric oxygen concentration, e.g. under a nitrogen atmosphere.

In an example of a further processing step the container may be filled by means of the above-mentioned sterile filling process of passing a hollow filling needle through a puncturable part of the boundary of the container and introducing a liquid medicinal substance, then withdrawing the filling needle. In the case of the above-mentioned vials and syringes, the puncturable part of the container may be a vial closure, or the plunger or closure of a syringe.

It is believed that introducing a liquid material in this manner via a filling needle inserted through the wall of a container having an oxygen-depleted internal atmosphere is novel.

Preferably a process for introducing a liquid substance into a container is provided and comprises the steps of:
providing a container which is bounded by a boundary having a part which is puncturable by a filling needle, and being suitable for a process of introduction of a liquid content into the container by passing a filling needle through the puncturable part and then introducing the liquid content via the needle;
the container having an oxygen concentration within the interior of the container at a level below ambient atmospheric concentration;
passing a hollow filling needle through the puncturable part,
introducing a liquid substance into the container via the filling needle, then withdrawing the filling needle.

Then optionally the residual puncture hole through the boundary wall may be more completely sealed e.g. in a known manner by application of a cover, by locally heating the boundary wall in the vicinity of the puncture hole e.g. with a focused laser beam etc. In some cases natural elasticity of a boundary wall e.g. if made of an elastomer may be adequate to close the residual puncture hole.

Suitably the container in this aspect of the invention may comprise the above-mentioned vial, syringe or carpule as described above, and the puncturable part may be the above-mentioned closure or plunger.

The oxygen concentration within the container may be at an experimentally determined level to achieve a suitable reduction in oxidative degradation of the liquid substance. Suitable the oxygen concentration within the container is 1 % or less.

If the container is to be filled in the above described manner then the container is preferably enclosed within the envelope in a state suitable for filling, e.g. in an empty state or containing e.g. an additive for mixing with a liquid medicinal substance.

In the case of containers being the above-mentioned vials or syringes, the closure or plunger material is usually more oxygen-permeable than vial or syringe barrel wall materials such as glass or COC. Therefore after this filling step, the closure or plunger material which is exposed to the atmosphere may be covered with a cover part made of a less oxygen-permeable or oxygen-impermeable material to prevent atmospheric oxygen diffusing back through the closure material into the interior of the container.

For example a vial may be provided with a clamp part to hold the closure sealingly in place in the mouth of the vial. Such a cover part may for example be attachable by a snap-fit attachment to the vial or such a clamp part. Such a cover part may be wholly or partly removable to allow access to the closure by a hollow needle which may be passed through the closure to extract liquid content from the vial for subsequent administration to a patient. An example of such a combination of clamp part and cover part is for example disclosed in WO-A-2004/018317. Suitable materials for such a cover part include polyethylene or polypropylene. The impermeability of such a cover part to oxygen may be enhanced by lamination with an oxygen-impermeable material such as metal foil.

After the container has been subjected to further processing such as the above-mentioned sterile filling step the further processed container is preferably stored in an environment with a reduced atmospheric oxygen concentration, for example a concentration as low as that within the container itself. By storing the container in such an environment the re-entry of oxygen into the container by diffusion back into the container may be inhibited. Such an environment may for example be provided by enclosing the container within an outer container bounded by an oxygen-impermeable material and enclosing an oxygen-absorbing material within the outer container. Such an outer container may also enclose an oxygen-reduced atmosphere such as an inert gas.

In another aspect the invention provides a combination product according to claim 13.

The oxygen concentration within the container may be below ambient atmospheric concentration. Suitably the concentration of oxygen in the atmosphere within the container is I % or less.

Such a container may be the above-mentioned vial or syringe

Preferably a container is provided, bounded by a wall having a puncturable part and/or having a mouth closed by a puncturable closure, 50% or more of the interior of the container being an atmosphere having an oxygen concentration below ambient atmospheric concentration. Preferably the container has an internal volume of which 75% or more is an atmosphere having an oxygen concentration below ambient atmospheric concentration. More preferably the container has an internal volume of which 90% or more is atmosphere having an oxygen concentration below ambient atmospheric concentration. Most preferably the container is empty except for this atmosphere having an oxygen concentration below ambient atmospheric concentration.

Such a container may be suitable for the above-described sterile filling process.

The oxygen concentration in the atmosphere within the container may be 1% or less.

Such a container may be the product of the above-described process for reduction of the atmosphere within the container to an oxygen concentration below ambient atmospheric concentration.

Such a container may be the above-mentioned vial or syringe.

The invention will now be illustrated by way of example only with reference to the accompanying drawings.
Figs. 1, 2, 3 and 4 illustrate the invention schematically.
Fig. 5 shows graphically the reduction of oxygen concentration in a vial.

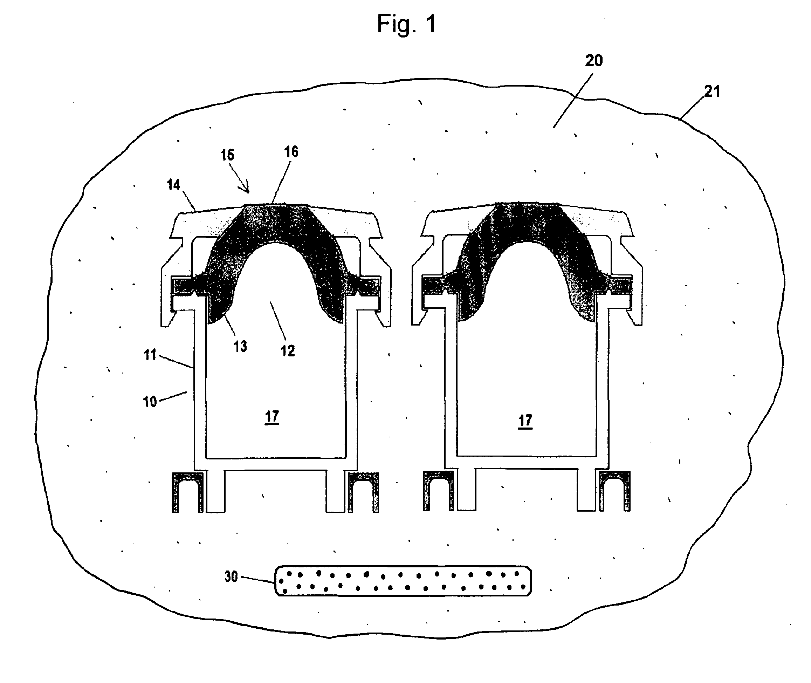

Referring to Fig. 1, this shows a schematic cross section through vials 10 (two are shown there may be more or less), enclosed within an envelope 20, with an oxygen-absorbing material 30 between the vials 10 and the envelope wall 21.

The vials 10 are of the type disclosed in WO-A-2004/018317, and are bounded by a boundary comprising a generally cylindrical vial body wall 11 having a mouth opening 12, closed by a closure 13, so that the combination of wall 11 and closure 13 forms the boundary. The wall 11 is made of a wall material, the COC polymer Topas 8007, Topas 6015 or Topas 6013, ca. 1.0 mm thick. Typically the volume of the vial is 1 - 100 ml. The closure 13 is made of an oxygen-permeable closure material, being an elastomer, for example as disclosed in WO-A-2005/014419. The closure 13 is held in place on vial 10 by means of a clamp part 14 snap-fitting under the flange around the rim of the mouth 12. The clamp part 14 has a central opening 15 through which part of the upper surface 16 of closure 13 is exposed to the outside. Vials as shown in Fig. 1 were provided by Aseptic Technologies, of Les Isnes, Belgium.

The atmosphere 17 inside vials 10 is oxygen-containing air from which microorganisms have been removed by filtration, achieved by assembling the combination of vial 10 and closure 13 in such an atmosphere.

Envelope 20 is bounded by flexible wall 21 of a multi-layer aluminium foil - polymer laminate, aluminium foil being impermeable to oxygen, closed around vials 10 by a heat seal (not shown). The volume within envelope 20 is such that when reduced by 20%, the approximate volume of atmospheric air consisting of oxygen, the wall will not be stretched over the vials 10 to an extent likely to risk tearing of the wall 21 or hindrance to the movement of atmosphere within the envelope 20.

Within envelope 20 is an oxygen-absorbing material 30, comprising an oxygen-absorbing substance within a permeable-walled sachet. This material 30 may be of a known type. Within envelope 20 the vials 10 may be disposed or otherwise isolated from the material 30 to minimise any likelihood of contamination of the vial 10 by material 30.

The oxygen absorbing material 30 absorbs oxygen from the atmosphere within envelope 20, leaving non-oxygen atmospheric gases such as nitrogen within envelope 20. The materials of vial wall 11 and closure 13 are oxygen-permeable and oxygen diffuses through these materials, particularly through closures 13 out of vials 10 until the oxygen concentration of the atmosphere 17 within the vials 10 has reached a predetermined level below ambient atmospheric concentration. A suitable level may be determined experimentally.

If the atmosphere within envelope 20 is air, then preferably the volume within envelope 20 is a volume such that when reduced by 20%, being the approximate volume of atmospheric air consisting of oxygen, the wall will not be stretched over the vials 10 to an extent likely to risk tearing of the wall 21 or hindrance to the movement of atmosphere within the envelope 20. Alternatively the envelope 20 may enclose a reduced-oxygen atmosphere e.g. a substantially oxygen-free inert gas such as nitrogen, so that the oxygen absorbing material 30 absorbs substantially only the oxygen that diffuses out of the vials 10. Alternatively the wall 21 may be rigid and the interior of the envelope 20 may be pressurized to atmospheric or above with an inert gas e.g. nitrogen.

Known oxygen-absorbing materials 30 can reduce the concentration of oxygen within envelope 20 to ca. 0.1 %, even from an initial air atmosphere. After a suitable length of time the concentration of oxygen in the atmosphere 17 within vial 10 may be reduced to a similar level.

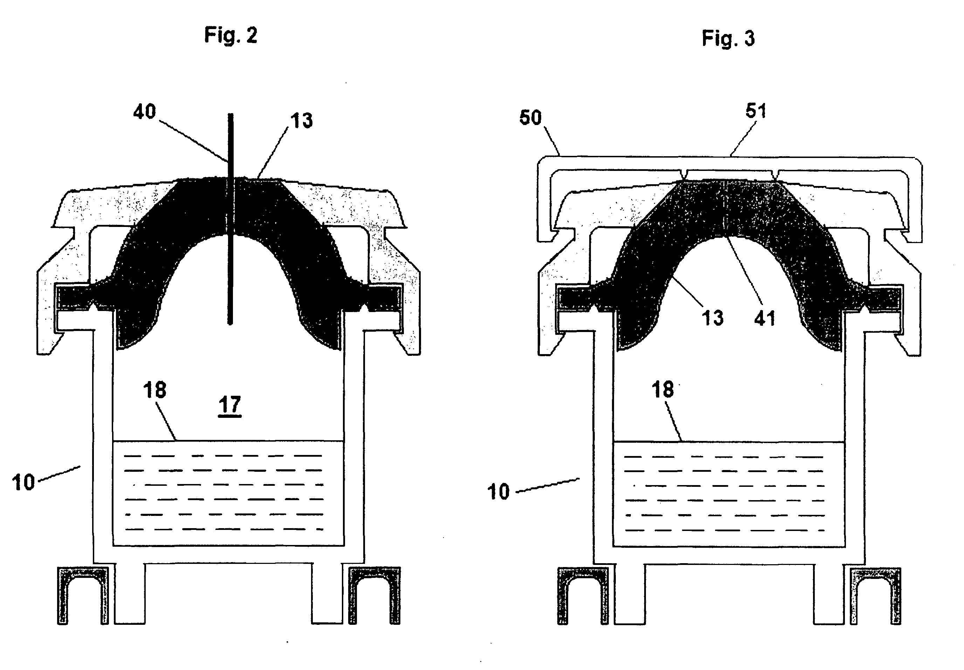

Vials 10 may be stored enclosed within envelope 20 to prevent re-entry of atmospheric oxygen into the vials 10. Alternatively after removal from envelope 20 the vials 10 may be stored under an inert atmosphere in an airtight container. The vials 10 are empty, so vials 10 may be removed from envelope 20 and subjected to a further processing operation as illustrated in Figs. 2 and 3.

As seen in Fig. 2 the empty vial 10 has been removed from envelope 20, a hollow filling needle 40 has been inserted through closure 13, and liquid content 18 has been introduced into the vial 10 through the needle 40.

As seen in Fig. 3 the needle 40 has been withdrawn from closure 13, leaving a residual puncture hole 41 which may be sealed by local heating of the outer surface of the closure 13 in the vicinity of the puncture hole 41, e.g. with a focused laser beam.

Fig. 3 also shows a cover part 50 attached by a snap-fit attachment to the clamp part 14. The cover part 50 is made of a resilient plastics material which is less oxygen-permeable than the elastomer material of closure 13, and covers the central opening 15 through which part of the upper surface 16 of closure 13 is exposed to the outside. A central part 51 of cover part 50 is removable e.g. by frangible connections (not shown) to the remainder of the cover part 50 to allow access to the closure 13 by a hollow needle (not shown) which may be passed through the closure 13 to extract liquid content 18 from the vial 10.

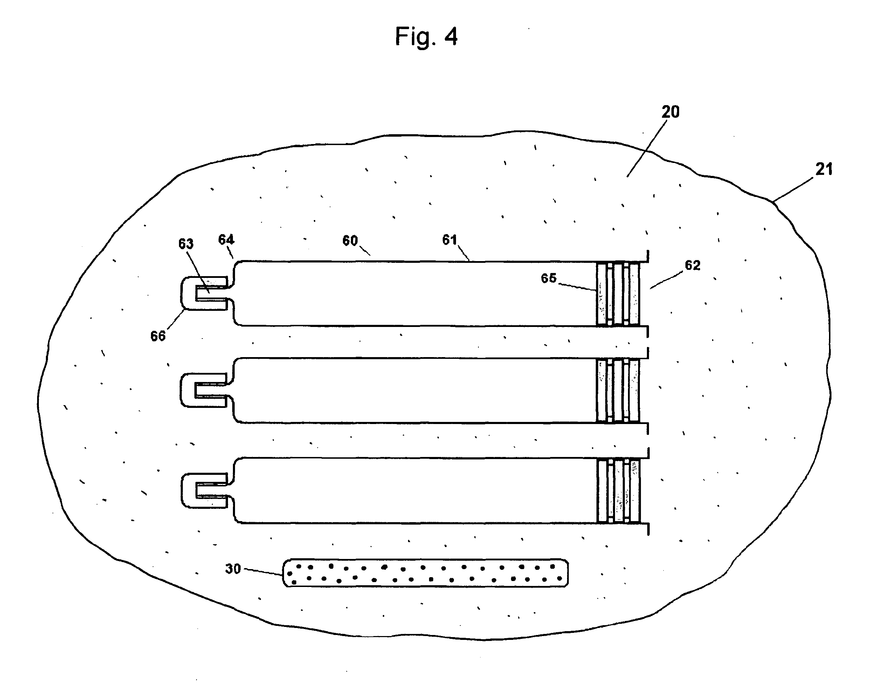

Referring to Fig. 4 Fig. 1 shows a schematic cross section through plural syringes 60 (three are shown there may be more or less), enclosed within an envelope 20, with an oxygen-absorbing material 30 between the syringes 60 and the envelope wall 21.

The syringes 60 have a generally cylindrical barrel wall 61 made of the COC polymer Topas 8007, Topas 6015 or Topas 6013, ca. 1.0 mm thick. Each syringe barrel 60 has an open end 62 and a nozzle 63 at an opposite nozzle end 64. Open end 62 is closed by a plunger 65 of an oxygen-permeable elastomer as disclosed in WO-A-2005/014419, making a sliding air-tight fit against the inside of barrel 61. Nozzle 63 is closed by a closure 66 also made of an oxygen-permeable elastomer as disclosed in WO-A-2005/014419.

Envelope 20 is bounded by flexible wall 21 of a multi-layer aluminium foil - polymer laminate, aluminium foil being impermeable to oxygen, closed around vials 10 by a heat seal (not shown). The volume within envelope 20 is such that when reduced by 20%, the approximate volume of atmospheric air consisting of oxygen, the wall will not be stretched over the syringes 60 to an extent likely to risk tearing of the wall 21 or hindrance to the movement of atmosphere within the envelope 20.

Within envelope 20 is an oxygen-absorbing material 30 as above. Within envelope 20 the syringes 60 may be disposed or otherwise isolated from the material 30 to minimise any likelihood of contamination of the vial 60 by material 30.

The oxygen absorbing material 30 absorbs oxygen from the atmosphere within envelope 20, leaving non-oxygen atmospheric gases such as nitrogen within envelope 20. The materials of barrel wall 61, plunger 65 and closure 66 are oxygen-permeable and oxygen diffuses through these materials out of syringes 60 until the oxygen concentration within the syringes 60 has reached a predetermined level below ambient atmospheric concentration. A suitable level may be determined experimentally.

Syringes 60 may be stored enclosed within envelope 20 to prevent re-entry of atmospheric oxygen into the vials 60. The syringes 60 are empty, so syringes 60 may be removed from envelope 20 and subjected to a further processing operation, analogous to the filling operation illustrated in Figs. 2 and 3, a filling needle (not shown) being passed through plunger 65 or closure 66.

In an experimental example three vials having a construction as shown in Fig. 1, being a product of the above-mentioned company Aseptic Technologies S.A., of Belgium. The vial was cylindrical, ca. 2.5 cm long, ca. 1.5 cm internal diameter, with Topas 6013 walls ca. 1.0 mm thick, the internal volume being ca. 4 ml. The open mouth of the vial was closed with a closure made of an elastomer made according to any of the Examples I to 4 of WO-A-2005/014419, with a thickness of ca. 2 mm. The vial was made by a sterile manufacturing process as described in WO-A-2005005128, leaving it with a sterile interior containing the normal ambient oxygen concentration of ca. 21 % v:v. The vial was enclosed in a flexible airtight envelope made of a plastics material - aluminium foil laminate. A commercially available oxygen absorbing material purporting to reduce atmospheric oxygen to ca. 0.1% v:v was positioned between the envelope and the vial, and the assembly of vial, envelope and oxygen-absorbing material was left. After two weeks the envelope was opened and a sample of the atmosphere inside the vials was withdrawn and sampled. It was found that the concentration of oxygen in the atmosphere within the vials was 13% v:v, i.e. reduced below the initial concentration.

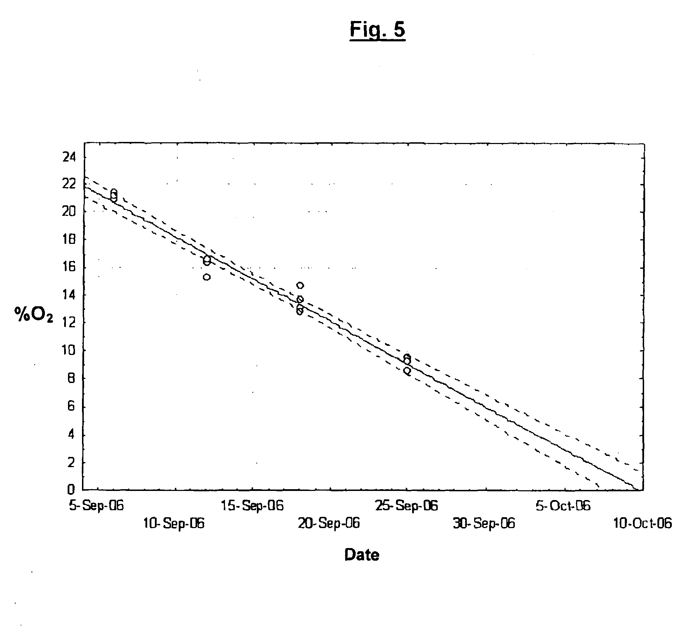

Fig. 5 shows graphically the reduction in oxygen concentration within the vials 10 from the original atmospheric ambient ca. 22%. It is seen that the reduction in oxygen concentration is effectively linear, extrapolated to reduce to near zero within ca. 5 weeks. In practice, on continuing to leave the vials within the envelope, after two months the residual oxygen concentration in the atmosphere within the vials was 0.4%.

The vials having been subjected to this oxygen reduction process were then subjected to a sterile filling process as described above, using a commercial filling line as provided by the company Aseptic Technologies SA, and filling the vials using a conventional filling needle etc. to a volume as used conventionally, under a conventional atmosphere of filtered air. It was found that the headspace above the liquid in the vials was ca. 0.65% oxygen.

As a control experiment vials were flushed in a conventional manner with nitrogen before their closures were fitted into their mouths. This was found to result in a variable concentration of around 5% of oxygen in the vials. Therefore the process of the invention is shown to have advantage over this conventional process.

## Claims

1. A process for the provision of a closed container (10) having an internal atmosphere (17) containing a lower concentration of oxygen than ambient atmospheric comprising:
providing a container (10) which is bounded by an oxygen-permeable boundary having a part (13) which is puncturable by a filling needle, and being suitable for a process of introduction of a liquid content into the container (10) by passing a filling needle (40) through the puncturable part (13) and then introducing the liquid content (18) via the needle (40) then withdrawing the needle (40), the container (10) having an internal volume of which 50% or more is an oxygen-containing atmosphere and being empty of liquid content
enclosing the container (10) within an envelope (20) bounded by an envelope wall (21) of a material which is less oxygen permeable than the wall material;
exposing the atmosphere between the container and the envelope wall to an oxygen-absorbing material (30) for a time such that the oxygen concentration of the atmosphere within the container is reduced below the initial concentration.

2. A process according to claim 1, further comprising the steps of :
removing said container (10) from said envelope (20);
inserting said filling needle (40) through said puncturable part (13);
introducing said liquid content (18) into said container (10) via said needle (40).

3. A process according to any one of claims 1 and 2, **characterised in that** the boundary comprises a boundary wall (11) having an opening (12) therethrough into the interior of the container (10) and which is closed by an oxygen-permeable puncturable closure material (13).

4. A process according to claim 3 **characterised in that** the container (10) comprises a vial comprising a vial body bounded by a body wall and having a mouth closed by a puncturable vial closure.

5. A process according to any one of claims 1 and 2, **characterised in that** the container (10) is a syringe barrel having a nozzle end and an open end opposite its nozzle end, the open end closed by a puncturable plunger, or an opening at the nozzle end of the syringe, and closed by an oxygen-permeable puncturable closure.

6. A process according to claim 4 or 5 **characterised in that** the vial or syringe has a vial wall or barrel wall which is made of an oxygen-permeable plastics material.

7. A process according to claim 6 **characterised in that** the oxygen-permeable plastics material comprises a COC polymer.

8. A process according to any one of the preceding claims **characterized in that** the container (10) contains an additive for mixing with a liquid substance to be introduced into the container (10).

9. A process according to any one of the preceding claims **characterized in that** the envelope wall (21) is a flexible wall.

10. A process according to any one of the preceding claims **characterized in that** the oxygen concentration of the atmosphere within the container (10) is reduced to 1 % or less.

11. A process according to any one of the preceding claims **characterized in that** a container (10) having an open mouth (12) and a closure (13) for the mouth are separately made, at least one of the container wall (11) or closure (13) being oxygen permeable;
the container (10) and closure (13) are assembled together to thereby enclose an oxygen-containing atmosphere comprising 50% or more of the internal volume of the container;
then the assembled container (10) and closure (13) are enclosed within an envelope (20) bounded by an envelope wall (21) of a material which is less oxygen permeable than both the wall and closure material;
the atmosphere between the container (10) and the envelope wall (21) is exposed to an oxygen-absorbing material (30) for a time such that the oxygen concentration of the atmosphere within the container (10) is reduced below the initial concentration.

12. A process according to any one of the preceding claims **characterized by** being followed by the container (10) being filled by means of a filling process of passing a hollow filling needle (40) through a puncturable part (13) of the container (10) and introducing a liquid medicinal substance (18), then withdrawing the filling needle (40).

13. A combination comprising:
a closed container (10) made at least in part of an oxygen-permeable wall material;
an envelope (20) bounded by an envelope wall (21) of an envelope material which is less oxygen permeable than the container wall material and enclosing the container (10);
an oxygen-absorbing material (30) between the container (10) and the envelope wall (21);
the container (10) comprising a puncturable wall part (13);
charaterized in that
the container (10) within the envelope (20) has an internal volume of which 50% or more is an oxygen-containing atmosphere and is empty of liquid content.

14. A combination according to claim 13, **characterised in that** the container (10) comprises a vial having a puncturable closure and being empty of liquid material.

15. A combination according to claim 13 or 14, **characterised in that** the oxygen concentration within the container (10) is 1% or less.

16. Use of a combination according to any one of claims 13 to 15, for use in a process for the provision of a closed container having an internal atmosphere containing a lower concentration of oxygen than ambient atmospheric.

17. Use of a combination according to claim 16, for use in a process according to any one of claims 1 to 12.

## Patentansprüche

1. Ein Verfahren zur Bereitstellung eines geschlossenen Behälters (10), der eine innere Atmosphäre (17) hat, welche eine niedrigere Sauerstoffkonzentration enthält, als die umgebende Atmosphäre, welches Folgendes umfasst:
Bereitstellung eines Behälters (10), der durch eine sauerstoffdurchlässige Begrenzung begrenzt ist, die einen Teil (13) aufweist, der durch eine Füllnadel durchstechbar ist und für ein Verfahren der Einbringung eines flüssigen Inhalts in den Behälter (10) geeignet ist, indem eine Füllnadel (40) durch den durchstechbaren Teil (13) eingeführt wird und dann der flüssige Inhalt (18) über die Nadel eingebracht wird und danach die Nadel (40) herausgezogen wird, wobei der Behälter (10) ein inneres Volumen hat, von dem 50 % oder mehr eine sauerstoffhaltige Atmosphäre ist und keinen flüssigen Inhalt aufweist;
Umschließung des Behälters (10) in einer Umhüllung (20), die durch eine Umhüllungswand (21) aus einem Material begrenzt ist, die weniger sauerstoffdurchlässig als das Wandmaterial ist;
Exposition der Atmosphäre zwischen dem Behälter und der Umhüllungswand gegenüber einem Sauerstoff absorbierenden Material (30) während einer solchen Zeit, dass die Sauerstoffkonzentration der Atmosphäre im Behälter unter die ursprüngliche Konzentration gesenkt wird.

2. Ein Verfahren nach Anspruch 1, welches ferner folgende Schritte umfasst:
Entnahme des erwähnten Behälters (10) aus der erwähnten Umhüllung (20);
Einführung der erwähnten Füllnadel (40) durch den erwähnten durchstechbaren Teil (13);
Einbringung des erwähnten flüssigen Inhalts (18) in den erwähnten Behälter (10) über die erwähnte Nadel (40).

3. Ein Verfahren nach irgendeinem der Ansprüche 1 und 2, **dadurch gekennzeichnet, dass** die Begrenzung eine Begrenzungswand (11) mit einer Öffnung (12) dadurch in das Innere des Behälters (10) umfasst, die durch ein sauerstoffdurchlässiges durchstechbares Verschlussmaterial (13) verschlossen ist.

4. Ein Verfahren nach Anspruch 3, **dadurch gekennzeichnet, dass** der Behälter (10) ein Fläschchen umfasst, welches einen Fläschchenkörper umfasst, begrenzt durch eine Körperwand, und welches mit einer Öffnung versehen ist, die durch einen durchstechbaren Fläschchenverschluss verschlossen ist.

5. Ein Verfahren nach irgendeinem der Ansprüche 1 und 2, **dadurch gekennzeichnet, dass** der Behälter (10) ein Spritzenkörper mit einem Düsenende und einem offenen Ende gegenüber seinem Düsenende ist, wobei das offene Ende durch einen durchstechbaren Kolben verschlossen ist, oder eine Öffnung am Düsenende der Spritze, und verschlossen durch einen sauerstoffdurchlässigen durchstechbaren Verschluss.

6. Ein Verfahren nach Anspruch 4 oder 5, **dadurch gekennzeichnet, dass** das Fläschchen oder die Spritze eine Fläschchenwand oder eine Körperwand hat, welche aus einem sauerstoffdurchlässigen Kunststoffmaterial hergestellt ist.

7. Ein Verfahren nach Anspruch 6, **dadurch gekennzeichnet, dass** das sauerstoffdurchlässige Kunststoffmaterial ein COC-Polymer umfasst.

8. Ein Verfahren nach irgendeinem der vorangegangenen Ansprüche, **dadurch gekennzeichnet, dass** der Behälter (10) einen Zusatzstoff zur Vermischung mit einer flüssigen Substanz enthält, die in den Behälter (10) eingebracht werden soll.

9. Ein Verfahren nach irgendeinem der vorangegangenen Ansprüche, **dadurch gekennzeichnet, dass** die Umhüllungswand (21) eine flexible Wand ist.

10. Ein Verfahren nach irgendeinem der vorangegangenen Ansprüche, **dadurch gekennzeichnet, dass** die Sauerstoffkonzentration der Atmosphäre im Behälter (10) auf 1 % oder weniger gesenkt ist.

11. Ein Verfahren nach irgendeinem der vorangegangenen Ansprüche, **dadurch gekennzeichnet, dass** ein Behälter (10) mit einer offenen Öffnung (12) und ein Verschluss (13) für die Öffnung getrennt hergestellt sind, wobei zumindest Behälterwand (11) oder Verschluss (13) sauerstoffdurchlässig ist;
der Behälter (10) und der Verschluss (13) zusammengefügt werden, um dadurch eine sauerstoffhaltige Atmosphäre zu umschließen, die 50 % oder mehr des inneren Volumens des Behälters umfasst;
danach der zusammengefügte Behälter (10) und Verschluss (13) in einer Umhüllung (20) umschlossen werden, die durch eine Umhüllungswand (21) aus einem Material begrenzt ist, das weniger sauerstoffdurchlässig als das Wand- und das Verschlussmaterial ist;
die Atmosphäre zwischen dem Behälter (10) und der Umhüllungswand (21) einem Sauerstoff absorbierenden Material (30) während einer solchen Zeit ausgesetzt wird, dass die Sauerstoffkonzentration der Atmosphäre im Behälter (10) unter die ursprüngliche Konzentration gesenkt wird.

12. Ein Verfahren nach irgendeinem der vorangegangenen Ansprüche, **dadurch gekennzeichnet, dass** folgt, dass der Behälter (10) mittels eines Füllverfahrens gefüllt wird, in dem eine hohle Füllnadel (40) durch einen durchstechbaren Teil (13) des Behälters (10) geführt wird und eine flüssige medizinische Substanz (18) eingebracht wird, wonach die Füllnadel (40) herausgezogen wird.

13. Eine Kombination, welche Folgendes umfasst:
einen geschlossenen Behälter (10), hergestellt zumindest teilweise aus einem sauerstoffdurchlässigen Wandmaterial;
eine Umhüllung (20) begrenzt durch eine Umhüllungswand (21) aus einem Umhüllungsmaterial, das weniger sauerstoffdurchlässig als das Behälterwandmaterial ist und den Behälter (10) umschließt;
ein Sauerstoff absorbierendes Material (30) zwischen dem Behälter (10) und der Umhüllungswand (21);
den Behälter (10), der einen durchstechbaren Wandteil (13) umfasst;
**dadurch gekennzeichnet, dass**
der Behälter (10) mit der Umhüllung (20) ein inneres Volumen hat, von dem 50 % oder mehr eine sauerstoffhaltige Atmosphäre ist und das keinen flüssigen Inhalt umfasst.

14. Eine Kombination nach Anspruch 13, **dadurch gekennzeichnet, dass** der Behälter (10) ein Fläschchen umfasst, das einen durchstechbaren Verschluss hat und kein flüssiges Material umfasst.

15. Eine Kombination nach Anspruch 13 oder 14, **dadurch gekennzeichnet, dass** die Sauerstoffkonzentration im Behälter (10) 1 % oder weniger beträgt.

16. Einsatz einer Kombination nach irgendeinem der Ansprüche 13 bis 15, zum Einsatz in einem Verfahren für die Bereitstellung eines geschlossenen Behälters, der eine innere Atmosphäre hat, die eine niedrigere Sauerstoffkonzentration als die umgebende Atmosphäre aufweist.

17. Einsatz einer Kombination nach Anspruch 16, zum Einsatz in einem Verfahren nach irgendeinem der Ansprüche 1 bis 12.

## Revendications

1. Procédé de production d'un récipient (10) fermé ayant une atmosphère intérieure (17) contenant une concentration d'oxygène inférieure à la concentration atmosphérique ambiante, comprenant :
produire un récipient (10) qui est limité par une surface de contour perméable à l'oxygène ayant une partie (13) qui peut être perforée par une aiguille de remplissage, et étant appropriée pour un procédé d'introduction d'un contenu liquide dans le récipient (10) en faisant passer une aiguille de remplissage (40) à travers la partie perforable (13) et puis en introduisant le contenu liquide (18) par l'intermédiaire de l'aiguille (40) puis en retirant l'aiguille (40), le récipient (10) ayant un volume intérieur dont 50 % ou plus sont une atmosphère contenant de l'oxygène et sont exempts de contenu liquide ;
enfermer le récipient (10) dans une enveloppe (20) limitée par une paroi (21) d'enveloppe en un matériau qui est moins perméable à l'oxygène que le matériau de paroi ;
exposer l'atmosphère entre le récipient et la paroi d'enveloppe à un matériau absorbant l'oxygène (30) pendant un temps tel que la concentration d'oxygène de l'atmosphère dans le récipient est abaissée en dessous de la concentration initiale.

2. Procédé selon la revendication 1, comprenant en outre les étapes consistant à :
retirer ledit récipient (10) de ladite enveloppe (20) ;
insérer ladite aiguille de remplissage (40) à travers la partie perforable (13) ;
introduire ledit contenu liquide (18) dans ledit récipient (10) par l'intermédiaire de ladite aiguille (40).

3. Procédé selon l'une quelconque des revendications 1 et 2, **caractérisé en ce que** la surface de contour comprend une paroi de séparation (11) contenant une ouverture (12) menant à l'intérieur du récipient (10) et qui est fermée par un matériau (13) de fermeture perforable perméable à l'oxygène.

4. Procédé selon la revendication 3, **caractérisé en ce que** le récipient (10) consiste en une fiole consistant en un corps de fiole limité par une paroi de corps et ayant une bouche fermée par une fermeture de fiole perforable.

5. Procédé selon l'une quelconque des revendications 1 et 2, **caractérisé en ce que** le récipient (10) est un fût de seringue ayant une extrémité à busette et une extrémité ouverte opposée à son extrémité à busette, l'extrémité ouverte fermée par un piston perforable, ou une ouverture à l'extrémité à busette de la seringue, et fermée par une fermeture perforable perméable à l'oxygène.

6. Procédé selon la revendication 4 ou 5, **caractérisé en ce que** la fiole ou seringue a une paroi de fiole ou paroi de fût qui est réalisé dans un matériau plastique perméable à l'oxygène.

7. Procédé selon la revendication 6, **caractérisé en ce que** le matériau plastique perméable à l'oxygène consiste en polymère COC.

8. Procédé selon l'une quelconque des revendications précédentes, **caractérisé en ce que** le récipient (10) contient un additif à mélanger avec une substance liquide à introduire dans le récipient (10).

9. Procédé selon l'une quelconque des revendications précédentes, **caractérisé en ce que** la paroi (21) d'enveloppe est une paroi flexible.

10. Procédé selon l'une quelconque des revendications précédentes, **caractérisé en ce que** la concentration d'oxygène de l'atmosphère à l'intérieur du récipient (10) est réduite à 1 % ou moins.

11. Procédé selon l'une quelconque des revendications précédentes, **caractérisé en ce qu'**un récipient (10) ayant une bouche ouverte (12) et une fermeture (13) pour la bouche sont réalisés séparément, au moins une de la paroi (11) de récipient ou de la fermeture (13) étant perméable à l'oxygène ;
le récipient (10) et la fermeture (13) étant assemblés pour renfermer une atmosphère contenant de l'oxygène constituant 50% ou plus du volume intérieur du récipient ;
le récipient (10) et la fermeture (13) assemblés étant alors enfermés dans une enveloppe (20) limitée par une paroi (21) d'enveloppe en un matériau qui est moins perméable à l'oxygène que le matériau tant de paroi que de fermeture ;
l'atmosphère entre le récipient (10) et la paroi (21) d'enveloppe est exposée à un matériau absorbant l'oxygène (30) pendant un temps tel que la concentration d'oxygène de l'atmosphère dans le récipient est abaissée en dessous de la concentration initiale.

12. Procédé selon l'une quelconque des revendications précédentes, **caractérisé en ce que** pour suivre, le récipient (10) est rempli au moyen d'un procédé de remplissage consistant à faire passer une aiguille de remplissage (40) à travers une partie perforable (13) du récipient (10) et à introduire une substance médicinale liquide (18), puis à retire l'aiguille de remplissage (40).

13. Combinaison comprenant :
un récipient (10) fermé réalisé au moins en partie dans un matériau de paroi perméable à l'oxygène ;
une enveloppe (20) limitée par une paroi (21) d'enveloppe dans un matériau qui est moins perméable à l'oxygène que le matériau de paroi de récipient et enfermant le récipient (10) ;
un matériau absorbant l'oxygène (30) entre le récipient (10) et la paroi (21) d'enveloppe ;
le récipient (10) comprenant une partie de paroi perforable (13) ;
**caractérisée en ce que**
le récipient (10) à l'intérieur de l'enveloppe (20) a un volume dont 50 % ou plus sont une atmosphère contenant de l'oxygène et sont exempts de matériau liquide.

14. Combinaison selon la revendication 13, **caractérisée en ce que** le récipient (10) consiste en une fiole ayant une fermeture perforable et étant exempte de matériau liquide.

15. Combinaison selon la revendication 13 ou 14, **caractérisée en ce que** la concentration d'oxygène à l'intérieur du récipient (10) est de 1 % ou moins.

16. Utilisation d'une combinaison selon l'une quelconque des revendications 13 à 15 à utiliser dans un procédé pour la production d'un récipient fermé ayant une atmosphère intérieure contenant une concentration d'oxygène inférieure à la concentration atmosphérique ambiante.

17. Utilisation d'une combinaison selon la revendication 16 à utiliser dans un procédé selon l'une quelconque des revendications 1 à 12.
